# EUROPEAN PATENT APPLICATION

(11) **EP 2 620 160 A1**
(43) Date of publication of application: **31.07.2013**
(21) Application number: 11826552.9
(22) Date of filing: 07.09.2011
(51) Int. Cl.: A61K 45/00, A23G 4/00, A23L 1/30, A61K 8/97, A61K 36/48, A61P 1/02, A61P 31/04, A61Q 11/00

(54) **COMPOSITION FOR ORAL USE**

(30) Priority: 21.09.2010 JP 2010211023
(71) Applicant: Lotte Co., Ltd., Tokyo 160-0023 (JP)
(72) Inventor: TSUGANE, Takanori, Saitama-shi Saitama 336-8601 (JP); SAEKI, Yoji, Saitama-shi Saitama 336-8601 (JP)
(74) Representative: Blodig, Wolfgang
(86) International application number: PCT/JP2011/005002
(87) International publication number: WO 2012/039101

(57) **Abstract**

In the conventional methods for suppressing dental caries using antibacterial agents, enzyme inhibitors or antibiotics, exocellular polysaccharides in oral biofilm prevent the permeation of antibacterial agents, enzyme inhibitors, antibiotics, or the like, and it is thus difficult to render intended suppressive effects on dental caries. Further, use of antibacterial agents or the like involves a high risk of resistant bacteria emergence, and thus it is not preferable. Thus, there remains a need for the development of a safer and more effective method for suppressing dental caries by regulating biofilm formation caused by dental caries causative bacteria instead of controlling the dental caries causative bacteria. A composition which regulates a quorum sensing according to the present invention can provide the inhibition of the tooth decay biofilm formation.

## Description

### [Technical Field]

The present invention relates to oral compositions for suppressing biofilm formation which are useful for prevention of dental caries and, in particular, to an oral composition which uses a novel method for suppressing biofilm formation by utilizing quorum sensing of oral microorganisms.

### [Background Art]

A microorganism adhered to a surface of a material is not singly present but it forms a biofilm together with other microorganisms in the distinctive structure. The biofilm works beneficially for humans as can be seen in the use of immobilized microorganisms, while it is also revealed to be a cause of dental caries and food contamination and thus extensive studies have been conducted in recent years.

The oral biofilm is formed by 700 or more species of bacteria and 10⁸ or more bacteria exist in 1 mg. *Streptococci,* which take up the majority (20% to 40%) among these bacteria, form a biofilm under the dynamic interbacterial communication mediated by interbacterial active substances on an oral cavity surface. In particular, *Streptococcus mutans* (*S. mutans*) produces a glutinous exocellular polysaccharide to play a primary role in the pathogenic biofilm formation. The oral biofilm is known to cause dental caries and periodontal diseases and these diseases have been regarded as the microorganism infectious diseases caused by bacteria including *S*. *mutans.*

Conventional prophylactic approaches to dental caries were based on a leading principle that dental caries is suppressed by sterilizing *S. mutans* or inhibiting enzymes such as glucosyltransferase and the like for prevention of plaque formation. However, in reality, the permeation of antibacterial substances, enzyme inhibitory substances, antibiotics, or the like is prevented by the exocellular polysaccharides covering the biofilm surface in the dental caries and hence in many cases intended effects are not achieved. In addition, the use of antibacterial agents, enzyme inhibitors, antibiotics, or the like, is always next to the risk of resistant bacteria emergence. Although procedures for suppressing a biofilm by mechanical removal such as brushing or scaling have also been used, it is difficult by the current procedures to practice adequate oral care for the elderly who is in need of nursing care, because it is not easy to apply such a mechanical control of oral biofilm to them.

From these viewpoints, there remains a need for the development of non-traditional methods for removal of biofilm and prevention of tooth decay. For controlling the oral biofilm, it is desirable to practice continuously as a daily routine and it is thus effective to use oral compositions such as food products including gums, etc. or toothpastes.

The inhibition of quorum sensing is a candidate for a new oral biofilm removal method. Recently, it has been revealed that quorum sensing (QS; cell population density-dependent gene expression regulation system), the molecular mechanism of signal transduction system between bacteria, works on the biofilm formation and pathogenicity expression by *S. mutans* and the QS in *S. mutans* is controlled by Competence Stimulating Peptide (CSP), which is an autoinducer. Currently, the various studies on the regulation of biofilm formation and pathogenicity expression targeting such a QS have been attempted in expectation of developing preventive methods for microorganism infectious diseases including oral diseases, which, however, have not yet reached practical use.

For example, Non Patent Literature 1 discloses that a bacterium called *S. salivarius* inhibits the biofilm formation by *S. mutans* and CSP can be regulated by the expression of a specific gene. However, an introduction of microorganisms into oral cavities involves safety issues and gene expression regulation is often accompanied by difficulties. Under such circumstances, development of a safer and simpler method for inhibiting biofilm formation has been expected.

### [Citation List]

### [Non Patent Literature]

NPL 1: Oral Microbiology And Immunology, 2009 24(2): pp152-61

### [Summary of Invention]

### [Technical Problem]

In the conventional methods for suppressing dental caries using antibacterial agents, enzyme inhibitors, antibiotics, or the like, exocellular polysaccharides in the oral biofilm prevent the permeation of antibacterial agents, enzyme inhibitors, antibiotics, or the like, and it is thus difficult to render the intended dental caries suppressive effects. Further, the use of antibacterial agents, enzyme inhibitors, antibiotics, or the like, involves a high risk of resistant bacteria emergence, and hence it is not preferable. For this reason, an object of the present invention is to provide a safer and more effective oral composition for suppressing dental caries by regulating the biofilm formation caused by dental caries causative bacteria instead of controlling the dental caries causative bacteria.

### [Solution to Problem]

The present inventors have conducted extensive studies and found that a composition which regulates quorum sensing can provide the inhibition of the tooth decay biofilm formation, whereby the present invention has been accomplished.

### [Advantageous Effects of Invention]

The biofilm formation caused by dental caries causative bacteria is inhibited by the oral compositions of the present invention. These oral compositions can be added to food or drink products, pharmaceutical products, toothpastes, or the like, and then used safely to prevent or treat the tooth decay.

### [Brief Description of Drawings]

[Fig. 1]

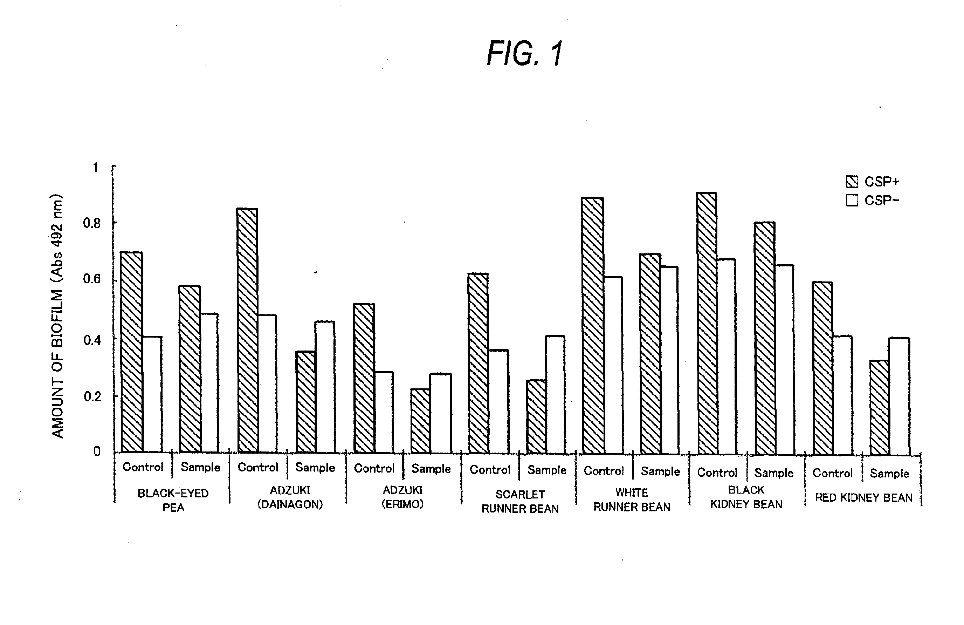

   Fig. 1 is a graph showing the biofilm formation amounts of the CSP-induced group and the CSP-uninduced group in each of the control group and in the sample group in Example 1.
[Fig. 2]

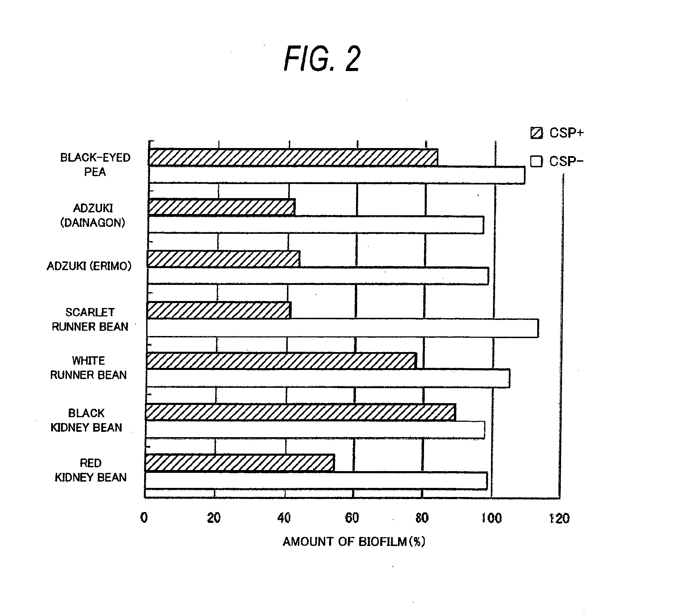

   Fig. 2 is a graph showing the rate of change in the biofilm formation amounts when a plant extract is added to each of the CSP-induced group and the CSP-uninduced group in Example 1.

### [Description of Embodiments]

Owing to the studies conducted so far as described in Non Patent Literature 1, quorum sensing (QS) of *S. mutans* is known to have been regulated by a specific microorganism or gene expression. However, in viewpoint of incorporation into an oral composition such as food products including gums or the like, and toothpastes for controlling dental caries biofilm as a daily routine, a number of problems remain to be unsolved in view of safety issues and convenience issues and thus any of these QS regulatory methods has not yet reached a practical use.

Under the circumstances, the present inventors conducted extensive studies and consequently found that quorum sensing of *S. mutans* can be regulated by a specific substance which is safely consumed every day, whereby the inhibition of dental caries biofilm formation by regulating quorum sensing was achieved.

More specifically, the present invention comprises inhibiting tooth decay biofilm formation associated with quorum sensing using an oral composition containing an extract of a bean.

According to the oral composition of the present invention, biofilm formation of CSP-dependent *S. mutans* is inhibited and thus pathogenic bacteria cannot adhere to oral cavities. Thus, suppression of biofilm without relying on mechanical procedures such as brushing or scaling can be achieved.

Also, unlike the conventional methods for suppressing dental caries which employ antibacterial agents, enzyme inhibitors, antibiotics, or the like, drawbacks of the difficulty to achieve the dental caries suppressive effects, due to the exocellular polysaccharides in the oral biofilm which prevent antibacterial agents, enzyme inhibitors, antibiotics, or the like, from being permeated does not arise.

Further, since the dental caries can be suppressed without using antibacterial agents, enzyme inhibitors, antibiotics, or the like, the risk of resistant bacteria emergence against antibacterial substances and antibiotics can be suppressed.

The kind of beans used in the present invention is preferably, but not particularly limited to, an extract extracted from at least one bean selected from black-eyed pea, adzuki bean, scarlet runner bean, white runner bean, black kidney bean and red kidney bean.

The method for obtaining the extract of a bean as an effective ingredient of the present invention include, but not particularly limited to, grinding of the seeds or fruits (beans) of the above bean plant by suitable grinding means and subsequent extraction such as a solvent extraction to prepare an extract. For the extraction solvent, one of water, lower alcohols such as methanol, ethanol, n-propanol, and n-butanol, and organic solvents such as ether, chloroform, ethyl acetate, acetone, glycerol, and propylene glycol, or a mixture of two or more thereof is used. Among them, a hot water or hydrophilic organic solvent is preferably used. Considering that the extract of the present invention is often used as a food or drink product, it is preferable that, from the aspect of safety, water and ethanol are used in combination to be the extraction solvent. The extraction is carried out preferably with 90% or less of ethanol, further preferably 60% or less of ethanol, further preferably 30% or less of ethanol, and most preferably with hot water.

For the extraction conditions, the extraction can be carried out at any of a high temperature, room temperature and a low temperature. It is preferred to extract at 50 to 90°C for about 1 to about 5 hours. The obtained extract may be filtered and, after distilling the extraction solvent, concentrated or freeze-dried under reduced pressure. Also, those obtained by fractionating and purifying these extracts using an organic solvent, column chromatography, or the like, can be used.

Further, since the oral composition of the present invention is very safe, it can be used every day when contained in oral compositions such as gargles, toothpastes, and mouth sprays; or drink or food products including confectioneries such as chewing gums, candies, tablet sweets, gummy jellies, chocolates, biscuits, and snacks; chilled sweets such as ice creams, sorbets, and ices; beverages, breads, pancakes, dairy products, processed meat products such as hams and sausages; processed fish meat products such as fish pastes and roasted fish pastes; delicatessens, puddings, soups and jams, etc.

The amount of the extract of a bean to be contained may vary depending on various production conditions, and it is preferably 0.01% by weight or more and 2.0% by weight or less, more preferably 0.01% by weight or more and 1.0% by weight or less with respect to the oral composition.

Since the present invention uses the extract which has been used for food for a long time, the safety thereof is not a problem even when it is introduced into the oral cavity or body. Also, the extract can be easily consumed when contained in a food product such as chewing gums, toothpastes, or the like, without requiring cumbersome procedures such as regulating gene expression and thus the biofilm can be continuously controlled every day.

### [Examples]

Hereinafter, the present invention is described with reference to Examples, but these Examples do not limit the scope of present invention.

### [Example 1]

### 1. Extract Preparation

7 Kinds of beans: black-eyed pea, adzuki bean (Dainagon), adzuki bean (Erimo adzuki bean), scarlet runner bean, white runner bean, black kidney bean and red kidney bean were used as plant samples.
Each of the plant samples was a commercially purchased product, and 20 g of which was finely ground using a grinder and extracted with 200 ml of water at 70°C for 2 hours. The obtained extract solution was centrifuged at 3000 rpm for 10 minutes, the supernatant was filtered and the freeze-dried product was subjected to following tests as hot water extracts of each plant.

### 2. Evaluation of suppressive activity on biofilm formation

### 2-1. Biofilm Formation

*S. mutans* UA159 strain was anaerobically incubated in 5 ml of Brain Heart Infusion (BHI) broth media at 37°C for 10 hours, the bacteria collected by the centrifugal separation at 3000 rpm for 10 minutes were treated with phosphate buffered saline (PBS) to prepare OD₅₅₀nm = 0.5 and used as a test sample suspension.
The biofilm formation was carried out by using a 96-well microplate. In each well, 60 µl of each plant hot water extract, 20 µl of CSP, 20 µl of a suspension containing *S. mutans* to be tested and 100 µl of Todd Hewitt Broth with 0.1% sucrose added were added and the incubation was carried out for 16 hours under the conditions of 37 °C and 5% CO₂, to form biofilms of the CSP-induced group. The final concentration of CSP was 1 µM and the final concentration of hot water extract of each sample was 1 mg/ml.
For comparison, the incubation was carried out under the same conditions as above with the exception of not adding CSP, to form the biofilms of the CSP-uninduced group.
Further, to be used as controls, each of the CSP-induced group and the CSP-uninduced group was incubated under the same conditions as above, except that the sample hot water extracts were not added thereto.

### 2-2. Biofilm measurement

For each of the CSP-induced group and the CSP-uninduced group, the supernatant after the incubation was removed and each well was washed twice with PBS. After washing, a 0.25% safranine solution was added to each well, allowed to stand for 15 minutes, thereafter from which the excess safranine solution was removed and each well was washed twice with PBS. After washing, ethanol was added to each well, safranine stained with shaking for 30 minutes was eluted and an absorbance at 492 nm was measured using a microplate reader to determine an amount of the formed biofilm.
The results are shown in Fig. 1 and Fig. 2.
Fig. 1 shows the biofilm formation amounts of a group (the sample group) to which the incubation was carried out with the addition of the plant extract and the control group, with each group presenting the CSP-induced group and the CSP-uninduced group. Fig. 2 shows the rate of biofilm formation amount of the sample group calculated when the biofilm formation amount of the control group is 100, with each sample group presenting the CSP-induced group and the CSP-uninduced group.
Referring to Fig. 1, in the control groups, the biofilm formation amounts were increased in the CSP-induced groups compared to the CSP-uninduced groups. Biofilm formed by quorum sensing due to the CSP as an autoinducer was considered responsible for the increase.
In the sample groups, the biofilm formation amounts in the CSP-induced groups (black bar) were lower than those of the control groups, whereas the biofilm formation amounts in the CSP-uninduced groups (white bar) were not different from those of the control groups. This is also evident as referred in Fig. 2, where the CSP-induced groups (black bar) had lower biofilm formation amounts in the sample groups than those in the control groups (below 100%), whereas the CSP-uninduced groups (white bar) had the biofilm formation amounts in the sample groups which were not different from those in the control groups (about 100%). These findings suggested that quorum sensing of S. mutans with the autoinducer CSP was regulated by the plant extracts added to the sample groups, which results in that the amounts of biofilm formed did not increase.
Further, referring to Fig. 1, when the hot water extracts of Dainagon adzuki bean, Erimo adzuki bean, scarlet runner bean and red kidney bean were added to the CSP-induced groups and incubated (CSP+ in the sample group), the biofilm formation amounts were lower than those in the groups to which neither CSP nor the plant extract was added (CSP- in the control group).

Using the adzuki extract prepared in Example 1, a chewing gum, candy and toothpaste were prepared in the routine method. The formulations are shown below. it should be noted that these formulations do not limit the scope of the present invention product.

### [Example 2]

A chewing gum was prepared in accordance with the following formulation.

| | |
|---|---|
| Gum base | 20.0% by weight |
| Sugar | 55.0 |
| Glucose | 15.0 |
| Starch syrup | 9.0 |
| Flavor | 0.5 |
| Erimo adzuki bean hot water extract (Example 1) | 0.5 |
| | 100.0 |

### [Example 3]

A candy was prepared in accordance with the following formulation.

| | |
|---|---|
| Sugar | 50.0% by weight |
| Starch syrup | 34.0 |
| Flavor | 0.5 |
| Dainagon adzuki bean hot water extract (Example 1) | 0.5 |
| Water | Balance |
| | 100.0 |

### [Example 4]

A toothpaste was prepared in accordance with the following formulation.

| | |
|---|---|
| Calcium carbonate | 50.0% by weight |
| Glycerol | 20.0 |
| Carbooxymethyl cellulose | 2.0 |
| Sodium lauryl sulfate | 2.0 |
| Flavor | 1.0 |
| Saccharin | 0.1 |
| Erimo adzuki bean hot water extract (Example 1) | 1.0 |
| Chlorhexidine | 0.01 |
| Water | Balance |
| | 100.0 |

This application claims the priority to the Japanese Patent Application No. 2010-211023, filed on September 21, 2010; and the disclosure of which is hereby incorporated by reference as a part of the present application.

## Claims

1. An oral composition for suppressing biofilm formation, comprising a substance which regulates quorum sensing in *Streptococcus mutans* controlled by CSP.

2. The oral composition for suppressing biofilm formation according to Claim 1, wherein the substance which regulates quorum sensing is an extract of a bean.

3. The oral composition for suppressing biofilm formation according to Claim 1 or 2, wherein the extract of a bean is an extract of at least one bean selected from the group consisting of adzuki bean, black-eyed pea, white runner bean, scarlet runner bean, black kidney bean and red kidney bean.

4. The oral composition for suppressing biofilm formation according to any one of Claims 1 to 3, wherein the extract of a bean is an extract of at least one bean selected from the group consisting of Dainagon adzuki bean, Erimo adzuki bean, scarlet runner bean and red kidney bean.

5. The oral composition for suppressing biofilm formation according to any one of Claims 1 to 4, not comprising an antibacterial agent, an enzyme inhibitor or an antibiotic.
